# EUROPÄISCHE PATENTSCHRIFT

(11) **EP 0 588 249 B1**
(45) Veröffentlichungstag und Bekanntmachung des Hinweises auf die Patenterteilung: **22.11.1995**
(21) Anmeldenummer: 93114542.9
(22) Anmeldetag: 10.09.1993
(51) Int. Cl.: C07C 245/06, C07C 291/08

(54) **Verfahren zur Herstellung von N-Hydroxy-N'-diazeniumoxiden**
Process for the preparation of N-hydroxy-N'-diazeniumoxides
Procédé pour la préparation d'oxides de N-hydroxy-N'-diazenium

(30) Priorität: 18.09.1992 DE 4231296
(43) Veröffentlichungstag der Anmeldung: 23.03.1994
(73) Patentinhaber: BASF Aktiengesellschaft, 67063 Ludwigshafen (DE)
(72) Erfinder: Massonne, Klemens, Dr., D-6721 Westheim (DE); Fischer, Martin, Dr., D-6700 Ludwigshafen (DE)

(56) Entgegenhaltungen:
- DE-A- 1 543 375
- DE-B- 1 019 657

## Beschreibung

Die vorliegende Erfindung betrifft ein neues Verfahren zur Herstellung von N-Hydroxy-N'-diazeniumoxiden der allgemeinen Formel I 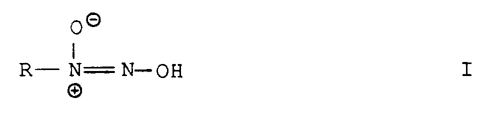
in der R für einen aliphatischen oder cycloaliphatischen Rest steht.

Aliphatische N-Hydroxy-N'-diazeniumoxide können nach der Lehre der DE-A 15 43 375 durch Photoreaktion der entsprechenden Aliphaten mit NO hergestellt werden.

Weiterhin führt nach der DE-A 10 19 657 die Umsetzung von aliphatischen Hydroxylaminen mit Alkalimetallnitriten zu den gesuchten Produkten. Hierbei fallen stöchiometrische Mengen an Alkalimetallsalzen als Nebenprodukte an.

Die JP-A 49/80023 lehrt die Umsetzung von substituierten Hydroxylaminen in organischen Lösungsmitteln mit Salzen oder organischen Estern der salpetrigen Säure. Auch hierbei entstehen stöchiometrische Mengen an Alkalimetallsalzen oder organischen Nebenprodukten.

Es bestand daher die Aufgabe, ein Verfahren zur Herstellung aliphatischer oder cycloaliphatischer N-Hydroxy-N'-diazeniumoxide zur Verfügung zu stellen, das die genannten Nachteile nicht aufweist.

Demgemäß wurde das oben definierte Verfahren gefunden, das dadurch gekennzeichnet ist, daß man ein Hydroxylamin der allgemeinen Formel II

R―NH―OH II

oder dessen Salz mit Nitrosylchlorid oder Nitrosylschwefelsäure in wäßriger Lösung umsetzt.

Das erfindungsgemäße Verfahren läßt sich schematisch folgendermaßen wiedergeben: 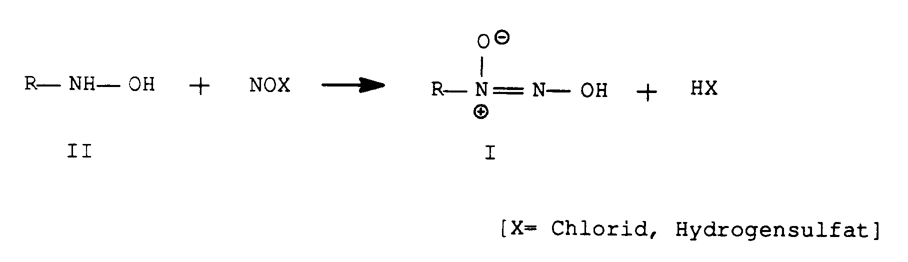
Die Ausgangsverbindungen II sind bekannt oder nach bekannten Methoden, etwa durch Reduktion entsprechender Oxime, zugänglich. Der Rest R steht für aliphatische und cycloaliphatische Reste. Diese Reste können unter den Reaktionsbedingungen inerte Substituenten wie Halogenatome tragen. Bevorzugt werden aber Alkyl- und Cycloalkylgruppen, insbesondere C₃-C₁₀-Alkyl wie n-Propyl, iso-Propyl, iso-Butyl oder Hexyl oder C₅-C₁₀-Cycloalkyl, wobei Cyclohexyl bevorzugt wird. Die Verbindungen II werden vorzugsweise in Form ihrer Salze, z.B. als Hydrochlorid oder Hemisulfat, eingesetzt. Als Nitrosoverbindungen dienen Nitrosylchlorid, vorzugsweise aber Nitrosylschwefelsäure. Die Nitrosylschwefelsäure liegt in der Regel als 5 bis 60 gew.-%ige, vorzugsweise 20 bis 50 gew.-%ige Lösung in Schwefelsäure vor.

Die Reaktion von II mit Nitrosylchlorid oder Nitrosylschwefelsäure wird in wäßriger Lösung, vorzugsweise in verdünnter Schwefelsäure, vorgenommen. Das Produkt kann in der Reaktionslösung direkt für Folgereaktionen eingesetzt werden. Da das Reaktionsprodukt in der Reaktionslösung aber nur kurzzeitig stabil ist, wird es in einer bevorzugten Ausführungsform daraus durch Extraktion mit einem organischen Lösungsmittel entfernt. Als Extraktionsmittel sind besonders aliphatische Kohlenwasserstoffe wie Pentan, Hexan und Heptan, cyclo-aliphatische Kohlenwasserstoffe wie Cyclohexan, aromatische Kohlenwasserstoffe wie Benzol, Toluol udn Xylol, halogenierte Kohlenwasserstoffe wie Methylenchlorid, Chloroform und Chlorbenzol sowie aliphatische Ether wie Diethylether und Methyl-tert.butylether zu nennen, die natürlich auch im Gemisch miteinander verwendet werden können. Die Menge des Extraktionsmittels wird zweckmäßigerweise so gewählt, daß man eine 0,1 bis 30 gew.-%ige, vorzugsweise 3 bis 10 gew.-%ige Lösung von I erhält.

In einer besonders bevorzugten Ausführungsform wird die Reaktion in Gegenwart eines Extraktionsmittels vorgenommen.

Bei der Reaktion gebildete Säure kann recyclisiert werden.

Die Temperatur liegt in der Regel zwischen dem Festpunkt der wäßrigen Reaktionsmischung (ca. -20°C) und 60°C.

Die Reaktion ist bei diesen Temperaturen im allgemeinen in weniger als einer Stunde beendet.

Das Verfahren kann sowohl kontinuierlich wie auch diskontinuierlich ausgeübt werden.

Da die Verbindungen I auch in der Extraktionslösung nur begrenzte Zeit stabil sind, schließt sich in der Regel an ihre Herstellung eine Reaktion zur Überführung in stabilere Salze nach bekannten Methoden an. Zur Salzbildung kommen z.B. Ammoniak, primäre Amine, Übergangsmetallsalze wie Kupfersulfat oder Nickelnitrat, vorzugsweise aber Kaliumhydroxyd in Betracht.

Das erfindungsgemäße Verfahren überführt Hydroxylamine in N-Hydroxy-N'-diazeniumoxide, ohne daß Salze oder organische Verbindungen als Nebenprodukte anfallen, und es erlaubt die Herstellung konzentrierter Lösungen des Produkts.

Die Verfahrensprodukte werden z.B. in Form ihrer Kupfersalze als Holzimprägnierungsmittel eingesetzt (DE-A 24 10 603).

### Beispiel

Zu 10,1 g (61 mmol) Cyclohexylhydroxylamin-Hemisulfat, 306 g 20 gew.-%iger Schwefelsäure und 270 g Toluol wurden bei 8°C 18,7 g (61 mmol) 41,7 gew.-%igen Nitrosylschwefelsäure in Schwefelsäure gegeben. Nach 20 Minuten wurde die organische Phase abgetrennt und mit 20 ml Wasser versetzt. Mit 50 %iger Kalilauge wurde der pH-Wert der wäßrigen Phase auf 11,5 eingestellt. Nach Extraktion wurden 33,4 g einer wäßrigen Phase isoliert, die 26,7 Gew.-% des Kaliumsalzes des N'-Cyclohexyl-N-hydroxy-diazenium-N'-oxids enthielt (49 mmol; 81 % Ausbeute).

## Patentansprüche

1. Verfahren zur Herstellung von N-Hydroxy-N'-diazeniumoxiden der allgemeinen Formel I 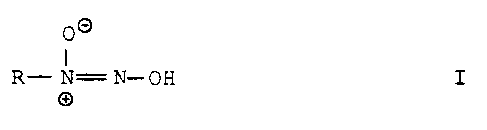 in der R für einen aliphatischen oder cycloaliphatischen Rest steht, dadurch gekennzeichnet, daß man ein Hydroxylamin der allgemeinen Formel II
R―NH―OH II
oder dessen Salz mit Nitrosylchlorid oder Nitrosylschwefelsäure in wäßriger Lösung umsetzt.

2. Verfahren nach Anspruch 1, dadurch gekennzeichnet, daß R für Cyclohexyl steht.

3. Verfahren nach Anspruch 1 oder 2, dadurch gekennzeichnet, daß man das N-Hydroxy-N'-diazeniumoxid I mit einem organischen Lösungsmittel aus der Reaktionslösung extrahiert.

4. Verfahren nach den Ansprüchen 1 bis 3, dadurch gekennzeichnet, daß man die Reaktion in Gegenwart eines organischen Extraktionsmittels vornimmt.

## Claims

1. A process for preparing N-hydroxy-N'-diazenium oxides of the general formula I 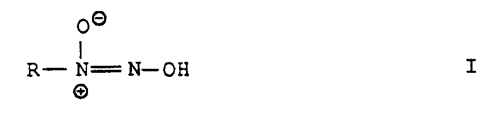 where R is an aliphatic or cycloaliphatic radical, which comprises reacting a hydroxylamine of the general formula II
R-NH-OH II
or its salt with nitrosyl chloride or nitrosylsulfuric acid in aqueous solution.

2. A process as claimed in claim 1, wherein R is cyclohexyl.

3. A process as claimed in claim 1 or 2, wherein N-hydroxy-N'-diazenium oxide I is extracted from the reaction solution using an organic solvent.

4. A process as claimed in any of claims 1 to 3, wherein the reaction is performed in the presence of an organic extracting agent.

## Revendications

1. Procédé de préparation d'oxydes de N-hydroxy-N'-diazénium de la formule générale I : 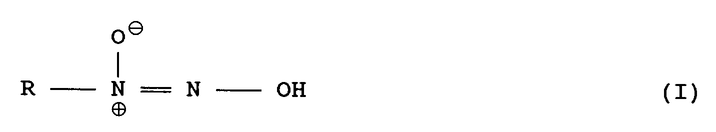 dans laquelle R représente un radical aliphatique ou cycloaliphatique, caractérisé en ce que l'on fait réagir une hydroxylamine de la formule générale II :
R ― NH ― OH (II)
ou un sel de celle-ci avec le chlorure de nitrosyle ou l'acide nitrosylsulfurique, en solution aqueuse.

2. Procédé suivant la revendication 1, caractérisé en ce que R représente le radical cyclohexyle.

3. Procédé suivant la revendication 1 ou 2, caractérisé en ce que l'on extrait l'oxyde de N-hydroxy-N'-diazénium I de la solution réactionnelle à l'aide d'un solvant organique.

4. Procédé suivant l'une quelconque des revendications 1 à 3, caractérisé en ce que l'on entreprend la réaction en présence d'un agent d'extraction organique.
